# EUROPEAN PATENT SPECIFICATION

(11) **EP 0 566 566 B1**
(45) Date of publication and mention of the grant of the patent: **06.12.1995**
(21) Application number: 91903101.3
(22) Date of filing: 10.01.1991
(51) Int. Cl.: A61K 31/565

(54) **COMPOSITIONS AND METHODS FOR ALLEVIATING MENOPAUSAL SYMPTOMS**
Zusammensetzungen und Verfahren zur Linderung der Symptome der Menopause
PROCEDES ET COMPOSITIONS DESTINES A REDUIRE LES SYMPTOMES LIES A LA MENOPAUSE

(43) Date of publication of application: 27.10.1993
(73) Proprietor: APPLIED MEDICAL RESEARCH, LTD., Fairfax, VA 22030 (US)
(72) Inventor: COHEN, Michael, NL-2443 CM Wassenaar (NL)
(74) Representative: Marchant, James Ian
(86) International application number: US9100235
(87) International publication number: WO9211855

(56) References cited:
- WO-A-88/07370

## Description

### Field of the Invention

This invention relates to a medicament for alleviating menopausal symptoms in women. More specifically, the invention relates to a medicament for alleviating climacterial menopausal and post-menopausal symptoms comprising an effective amount of a composition comprising melatonin and at least one estrogen and/or at least one androgen.

### Background of the Invention

The basic physiological and hormonal change associated with menopause in human females is a cessation of the cyclic follicular growth and maturation. A woman who is in menopause will not ovulate and will not have the associated menstrual bleeding which normally accompanies the ovulatory cycle. Menopause can begin physiologically as part of a woman's life cycle, signifying the end of her reproductive life cycle. A woman also can begin menopause as a result of the surgical removal of her ovaries or as a part of a pathological form of ovarian failure.

In menopause a woman's ovarian estradiol synthesis and secretion comes to a gradual end. As a result, about sixty percent of her natural estrogen production is stopped; the peripheral tissue aromatization of androstenedione, which originates in the ovaries and the adrenal cortex, continue to sustain the remaining forty percent in the form of estrone. The quantity of estrone which is produced during menopause, however, is inadequate to support the estrogen requirements of estrogen dependent target organs, such as the hypothalamus, pituitary, breasts, urogenital tract and bone metabolism, in many women.

The relative insufficiency of estrogen produces an atrophy of epithelia and a significant decrease in the blood supply to estrogen target organs. As a direct result of this phenomenon, women in menopause experience symptoms of instability of their hypothalamic thermoregulatory centers, and many suffer from recurrent symptoms known as "hot flashes." Women suffering from "hot flashes" can experience nervousness, insomnia, fatigue, and/or depression. Peripheral estrogenic target organs also are affected by the decline in plasma estradiol, and symptoms of vaginal irritation, dyspareunla, dysuria vaginal infections and pruritus are common.

The mineralization of bone matrix is also affected significantly by the estrogen deficiency, resulting in a pathological condition known as osteoporosis. This condition is associated with significant morbidity and mortality, primarily as a result of hip fractures, although other bone fractures also are common.

The medical community is in general agreement that, if estrogen could be administered without risk, all women in the menopausal phase of their life cycle should receive estrogen medication to counteract and abolish the significant health risks which result from estrogen deficiency. To date, however, there have been substantial risks associated with the administration of estrogen, such that only periodic and cautious medication with estrogen is advised.

Current regimens of estrogen medication typically utilize ethinyl estradiol or its methyl ether and/or conjugated estrogens. Dosages are kept as low as is practically possible to achieve some benefit with a minimum of risk. Typical dosages range from about 0.2 mg to about 1 mg daily.

As noted above, the administration of estrogen has been accompanied by many risks which have prevented their more common and widespread use. Potential risks include a higher risk of endometrial cancer, breast cancer and benign breast and liver tumors. Gallbladder disease also is more common among women who take estrogen, as are such systemic problems as hypertension and water retention.

To decrease the risk of developing any of these problems, it generally is recommended to administer estrogens in a cyclic fashion and to combine their administration with a progestin, which is thought to reduce the risks which are associated with long-term estrogen use.

In addition, it has been found that estrogen supplements do not lessen menopausal symptoms in some women unless given at very high doses. In some cases, such women have been given doses of an androgen, such as methyltestosterone, in place of estrogen supplementation, as a means of alleviating their symptoms. This type of therapy, however, also has been accompanied by the risk of developing significant negative side effects, including endometriosis and hyperplasia.

In view of the drawbacks and negative side effects of conventional estrogen supplementation and estrogen and androgen medication, new methods of alleviating menopausal and post-menopausal symptoms are sought. Accordingly, it is an object of this invention to provide a medicament for alleviating menopausal symptoms which is highly effective and provides the benefits and avoids the adverse effects associated with conventional estrogen therapy.

### Summary of the Invention

In accordance with the present invention, there is disclosed a medicament for alleviating or preventing menopausal and post-menopausal symptoms in women suffering from or susceptible to such symptoms which comprises administering effective dosages of melatonin in combination with one or more estrogens, and/or one or more androgens. As used herein, "menopausal and post-menopausal symptoms include, for example, atrophic vaginitis, kraurosis vulvae, hypogonadism, osteoporosis and vasomotor symptoms.

### Detailed Description of the Invention

Melatonin (N-acetyl-5-methoxytryptamine) is a hormone synthesized and secreted by the pineal gland. The exact role of the hormone has not yet been determined. Studies have shown that in rodent species the hormone has an inhibitory effect on the gonads and their development. The daily injection of melatonin into Syrian golden hamsters has had an inhibitory effect on the development of the gonads, the weight of the testes of males and on ovulation in females. Female rats injected with melatonin at certain times of the day also showed an inhibition of ovulation. Published results of studies wherein other mammalian species were injected with melatonin have not revealed similar results. Exogenous melatonin administration in humans has been studied in conjunction with a hypothesis that an abnormal melatonin rhythm is associated with endogenous depression and for pharmokinetic purposes (Waldhauser, Neuroendocrinology 39:307, 313 [1984]) and in connection with sleep-wake rhythms and the phenomenon of "jet-lag" following airplane trips associated with change in time zones.

The pineal gland undergoes a process of calcification which is directly related to age. Concomitant with this calcification, a significant decrease in serum melatonin has been reported. The present invention is based on the discovery that the administration of pharmacological doses of melatonin in combination with estrogen supplementation to menopausal women results in an alleviation of menopausal symptoms while minimizing the risks that have accompanied conventional estrogen therapy. The administration of melatonin increases the tolerance level for potent doses of estrogen.

As used herein, the term melatonin also encompasses melatonin analogs which, when administered in combination with an estrogen to a woman in menopause, increases the woman's tolerance for the estrogen. Such melatonin analogs can have the general formula: 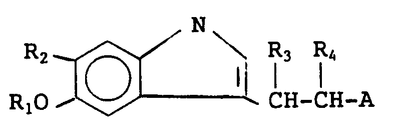
wherein R₁, R₃ and R₄, individually, are hydrogen or an alkyl group having 1 to 4 carbon atoms, R₂ is selected from hydrogen, hydroxy or an alkoxy group having from 1 to 4 carbon atoms, and A is either -OH or 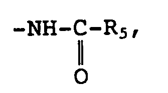
wherein R₅ is either hydrogen or an alkyl group having from 1 to 4 carbon atoms, provided that if A is 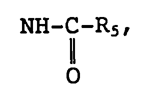
and R₁ and R₅ are both methyl and R₂ is hydrogen, both of R₃ and R₄ are not hydrogen. Preferred compounds are those in which R₂ is hydrogen or methoxy, with hydrogen being most preferred. Melatonin analogs encompassed within this definition include N-acetyl serotonin, N-acetyl-5-hydroxy-6-methoxytryptamine, 6-hydroxy-melatonin, 5-hydroxytryptophol and 5-methoxytryptophol, with N-acetyl serotonin being preferred.

The melatonin (or melatonin analog) desirably is administered in dosages sufficient to restore the patient's level of melatonin to her pre-menopausal limits or above.

As used herein the word estrogen refers both to conjugated estrogens, obtained directly from natural sources, and to synthetic estrogens. Conjugated estrogens include estrone, equilin, 17-α-dihydroequilin, 17-α-estradiol, equilenin and 17-α-dihydroequilenin. Typically, these products are administered as the salts of their sulfate esters. Preferred synthetic estrogens include ethinyl estradiol (i.e. 17α-ethynyl-3,17β-dihydroxy-estra-1,3,5(10)-triene) and mestranol (17α-ethynyl-17β-hydroxy-3-methoxy-estra-1,3,5(10) triene). Other useful synthetic estrogens include estradiol (3,17β-dihydroxyestra-1,3,5(10)-triene), estriol (3,16,17β-trihydroxyestra-1,3,5(10)-triene), estrone (3-hydroxy-estra-1,3,5(10)triene-17-one), diethylstilbestrol, quinestradiol (3-cyclopentyloxy-16α, 17β-dihydroxyestra-1,3,5(10)-triene) and estrone sulfate.

One embodiment of this invention is directed to compositions comprising melatonin and at least one estrogen. Depending upon the particular symptoms to be alleviated or prevented, the melatonin and estrogen can be administered to women orally, parenterally, in the form of an implant or as a vaginal cream or lotion. Administration is most convenient when the hormones are in oral dosage form, such as capsules, tablets, suspensions or solutions. Capsules or tablets are preferred. Capsules can be prepared by mixing the compound with a pharmaceutically-acceptable excipient and then filling gelatin capsule with the mixture in accordance with conventional procedures. Alternatively, the hormones can be mixed with one or more lubricants, such as stearic acid or magnesium stearate, flavor ameliorating agents, disintegrating elements, including potato starch, calcium phosphate tribasic and alginic acid, binders, such as gelatin, carnauba wax and corn starch, and/or tablet bases including lactose, corn starch, talc and sucrose, and then pressed into tablets.

As an alternative to oral administration, the melatonin and estrogen can be administered parenterally or in the form of a solid implant. For parenteral administration, the melatonin and estrogen are provided in injectable doses of a solution or suspension of the hormones in a physiologically acceptable diluent with a pharmaceutical carrier. The carrier can comprise water or an oil and optionally also can contain a surfactant or other pharmaceutically acceptable adjuvant. Suitable oils include those of animal, vegetable, petroleum or synthetic origin, including peanut, soybean, corn, sesame, castor and mineral oil. Preferred liquid carriers include water, saline, aqueous sugar solutions, and glycols such as propylene glycol or polyethylene glycol.

The melatonin and estrogen also can be administered in the form of an implant, which is formulated such that it will provide a sustained release of the hormones over time. To make the implant, the hormones can be compressed into small cylinders and placed inside a physiologically acceptable shell material, such as a biodegradable or porous polymer, in accordance with conventional implant technology.

Alternatively, the estrogen and melatonin can be provided in the form of a cream or lotion. The cream or lotion is prepared using conventional bases, such as glyceryl monostearate, propylene glycol monostearate, methyl stearate, phenylethyl alcohol, sodium lauryl sulfate, glycerin and/or mineral oil in accordance with procedures well-known in the art.

A number of regimens are suitable for administering a combination of melatonin and estrogen. The particular regimen selected is dependent upon such factors as the number and severity of the particular symptoms or conditions to be treated and the method of administration. Lower doses of the hormone typically are needed for administration by way of an injection or implant than for oral administration. Generally, the estrogen is administered in the range of 0.125 to 15 mg per day. The melatonin is administered in an amount sufficient to increase the recipient's physiological tolerance for estrogens and to reduce the negative side effects of conventional estrogen therapy to a minimal level. Generally, the melatonin is administered in the range of 1 to 2000 mg per day. The actual amount of estrogen and melatonin provided in each daily dose will depend upon the particular estrogen chosen, its relative potency, and the method of administration selected. For example, a lesser quantity of a more potent estrogen may achieve the same results as a larger quantity of a less potent estrogen. Synthetic estrogens generally are more potent than are natural estrogens. Thus, when a natural estrogen is used, the ratio of estrogen to melatonin generally will be higher than when a synthetic estrogen is used. In addition, lower dosages generally are needed for administration of an implant or intravenous or intramuscular injection than for oral administration. The hormones can be administered on a continuous basis or cyclically (i.e., one or both hormones is administered for a certain number of days and then withheld for a certain number of days), as illustrated in the particular suggested regimens set forth below.

In one example of a useful regimen, the melatonin and estrogen can be administered daily in oral form to women suffering from such conditions as atrophic vaginitis, kraurosis vulvae, or hypogonadism, for about 21 days, then for about the next 7 days either the melatonin is administered in the absence of the estrogen or both hormones are withheld. With either of these regimens, the cycle can be repeated as necessary. With these regimens, typically the estrogen is administered in a dose of 1 to 8 mg per day and the melatonin is administered in a dose of 3 to 2000 mg per day, preferably 30 to 300 mg per day.

In an alternative regimen, the estrogen and melatonin are orally administered for 60-90 days, then both hormones are withheld until the symptoms reappear. With this regimen, given the length of time of continuous estrogen administration, the estrogen typically is administered in lower dosages than in the regimens discussed above. Generally the estrogen is administered in the range of 0.3 to 1.25 mg per day and the melatonin is administered in the range of 30 to 300 mg per day.

To alleviate osteoporosis or vasomotor symptoms, a combination of melatonin and estrogen can be administered orally on a daily, chronic basis in amounts generally ranging from 0.125 to 2.5 mg estrogen per day and 3 to 2000 mg, preferably 30 to 300 mg, melatonin per day. Alternatively, the estrogen can be administered in the same general amounts for about 21 days in combination with the melatonin, then the estrogen is withheld for about the next 7 days. During this seven day period, the melatonin also can be withheld or its administration can be continued.

If the melatonin and estrogen are to be administered by intravenous or intramuscular injection, the hormones conveniently can be provided in the form of lyophilized cakes prior to being placed into solution and injected. For example, to prepare lyophilized cakes, 5 to 150 mg estrogen, preferably 20 to 30 mg estrogen, and 1 to 10 grams melatonin, preferably 3 to 7 grams melatonin, can be mixed with a conventional base or excipient, such as lactose or sodium citrate, and lyophilized. The cake then can be dissolved in a sterile diluent, such as sterile water or benzyl alcohol, to achieve the desired final hormone concentrations, and the pH of the solution can be adjusted, if necessary, to assure that the solution is physiologically acceptable with sodium hydroxide or hydrochloric acid. Individual daily doses then can be obtained from this master stock solution.

If the estrogen and melatonin are prepared in the form of a cream or lotion, as discussed above, each gram of the cream or lotion desirably contains 0.2 to 2.5 mg estrogen and 0.1 to 3 mg melatonin, preferably 0.6 mg estrogen and 0.5 mg to 3 mg melatonin. Administration of the hormones in this fashion is especially beneficial for the alleviation of symptoms of kraurosis and atrophic vaginitis. The cream or lotion is applied locally one to about ten times a day for as many days as needed. Ideally, the medication is applied about four times a day for about 21 days, then is withheld for about 7 days, the cycle being repeated as necessary.

In any of the foregoing embodiments, if desired, the melatonin and estrogen can be administered in combination with a progestogen. Progestogens have been administered with conventional estrogen therapy to lessen the risk of endometrial hyperplasia associated with the administration of estrogen and can be included in the compositions of this invention to achieve the same benefits. Any progestationally active compound is suitable for use as the progestogen component in the present invention. Suitable progestogens include progesterone and derivatives thereof. The presently preferred progestogen is norethindrone (i.e., 19-nor-17α-ethynyl-17β-hydroxy-4-androsten-3-one) and norgestrel (13β-ethyl-17α-ethynyl-17βhydroxygon-4-en-3-one). Other progestogens include chlormadinoneacetate (6-chloro-17-hydroxy-pregna-4,6-diene-3,20-dione acetate), norethynodrel (17α-ethynyl-17-hydroxyestr-5(10)-en), medroxyprogesterone acetate (17α-acetoxy-6α-methyl-pregn-4-ene-3,20-dione), megestrol acetate (17α-acetoxy-6-methyl-pregna-4,6-diene-3,20-dione), lynestrenol (17α-ethynyl-17β-hydroxy-estr-4-en-3-one), quingestrone (3-cyclopentyloxy-pregna-3,5-diene-20-one), norethindrone acetate (17β-acetoxy-17α-ethynyl-estr-4-en-3-one), ethynodiol acetate (3β,17β-diacetoxy-17α-ethynyl-estr-4-one), dimethisterone [17β-hydroxy-6α-methyl-17-(1-propynyl)-androst-4-en-3-one], and levonorgestrel.

In the regimens described above, the progestogen can be administered with the estrogen and melatonin or it can be administered for a desired period of time immediately following withdrawal of the estrogen. For example, if estrogen and melatonin are administered orally for 60-90 days, the progestogen can be administered in combination with the other hormones over the same period or it can be administered for 7-14 days following the estrogen-melatonin therapy. The amount of progestogen administered depends upon the strength of the particular progestogen chosen, the method of administration and the length of time of administration. Typically, the progestogen is administered in daily doses of 7.5 to 2500 »g, preferably 7.5 to 600 »g.

In an alternative embodiment of this invention, the melatonin is combined with an androgen in place of, or in combination with, an estrogen. Preferred androgens include methyltestosterone and fluoxymesterone. It has been found that some of the symptoms of menopause, such as vasomotor symptoms of estrogen deficiency, can be treated more effectively by including an androgen in the therapeutic regimen than by treating with estrogen alone or with a combination of estrogen and melatonin. Generally, the androgen is administered in a dose of 0.2 to 15 mg, preferably 0.5 to 2.5 mg, and the melatonin and estrogen are administered in the general ranges set forth above. This combination of hormones can be administered in acyclic fashion, as described above, or continuously, as needed.

In any of the suggested regimens set forth above, on those days that both the melatonin and one or more additional hormones are administered, they conveniently are combined and administered together, although they also can be administered separately.

In a preferred embodiment of this invention, the hormone compositions are administered in oral dosage form, preferably in the form of pills or capsules. The pills or capsules can be packaged in any manner suitable for proper delivery and use. If the pills are to be administered in accordance with a cyclic regimen, they preferably are packaged in the form of a kit or package in which the daily unit dosage forms are provided or arranged in a contiguous, sequential order which will enable the woman taking the pills to take the proper formulation on any given day. Suitable kits or packages include the conventional bubble plastic package used for oral contraceptives containing individual bubbles for either 21 or 28 pills, depending upon the regimen selected, in a sheet of flexible plastic. The bubbles are sealed by a sheet of plastic which can break and release a pill when the bubble is pressed. On the first day of medication, the first pill in the sequence is removed from the individual slot and taken. The next pill in the sequence is taken the next day and so on thereafter until the dispenser is empty. A new dispenser is begun 28 days after the first dispenser was begun. If a 21 day regimen is to be followed, an additional 7 pills, each containing only a placebo, can be included in the dispenser package, if desired, so that a pill is taken every day of the cycle.

In addition to alleviating various menopausal and post-menopausal symptoms, the compositions of this invention also can be administered on a preventive basis, especially to prevent osteoporosis. A woman found to be at risk can be given a composition in accordance with this invention, containing relatively low doses of the hormones, on a continuous, daily basis.

## Claims

1. Use of melatonin or a melatonin analog for the manufacture of a medicament for alleviating or preventing menopausal or post-menopausal symptoms in a woman suffering from or susceptible to such symptoms by a method which comprises administering said melatonin orally, intravenously or in the form of an implant on a daily basis in combination with one or more estrogens, one or more androgens or both an androgen and an estrogen.

2. Use according to claim 1, wherein the melatonin is administered in combination with one or more estrogens and the daily dosage of melatonin is sufficient to increase the female's physiological tolerance for the estrogen administered.

3. Use according to claim 2, wherein the daily dosage level of estrogen is from 0.125 mg to 15 mg, preferably from 0.3 mg to 8 mg, and the daily dosage level of the melatonin is from 1 mg to 2000 mg, preferably from 30 mg to 300 mg.

4. Use according to claim 2 or 3, wherein the estrogen is selected from estrone, equilin, 17-α-dihydroequilin, 17-α-estradiol, equilenin, 17-α-dihydroequilenin, ethinyl estradiol, mestranol, estradiol, estriol, diethylstilbestrol and quinestradiol optionally in the form of a sulfate ester salt.

5. Use according to any of claims 1 to 4, wherein
i) a combination of an estrogen and melatonin is administered daily for about 21 days followed by administration of melatonin daily for about 7 days in the absence of an estrogen; or
ii) a combination of an estrogen and melatonin is administered daily for about 21 days followed by about 7 days without melatonin or estrogen administration; or
iii) an estrogen and melatonin are administered for 60 to 90 days, then both the estrogen and melatonin are withheld until the menopausal or post-menopausal symptoms reappear.

6. Use according to any of claims 1 to 5, wherein the method of administration is by intravenous injection in a physiologically suitable carrier, by implant or orally.

7. Use according to any of claims 1 to 6, wherein a progestogen is administered in combination with the melatonin and one or more estrogens.

8. Use according to claim 7, wherein the progestogen is selected from the group consisting of norethindrone, norgestrel, chlormadionone-acetate, norethynodrel, medoxyprogesterone acetate, megesterol acetate, lynestrenol, quingestrone, norethindrone acetate, ethynodiol acetate, dimethisterone, and levonorgestrel.

9. Use according to any of claims 1 to 8, wherein the melatonin is administered in combination with at least one androgen.

10. Use according to claim 9, wherein the androgen is methyltestosterone or fluoxymesterone.

11. Use according to claim 9 or 10, wherein the daily dosage of androgen is from 0.2 mg to 15 mg and the daily dosage of melatonin is from 1 mg to 2000 mg.

12. Use of one or more estrogen and/or melatonin or a melatonin analog for the manufacture of medicaments for effecting estrogen supplementation in a menopausal or post-menopausal woman by a method which comprises administering orally, intravenously or in the form of an implant effective doses of one or more estrogens in combination with a sufficient amount of melatonin to increase the woman's physiological tolerance for estrogen.

13. Use of melatonin or a melatonin analog for the manufacture of a medicament for treating osteoporosis, preventing osteoporosis, treating atrophic vaginitis, treating kraurosis vulvae or treating vasomotor symptoms of estrogen deficiency by a method which comprises administering orally, intravenously or in the form of an implant melatonin in combination with at least one estrogen and/or at least one androgen.

14. Use according to any of claims 1 to 13, wherein the melatonin analog has the general formula 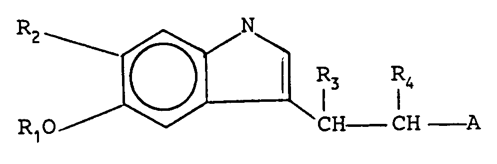 wherein R₁, R₃ and R₄, individually, are hydrogen or an alkyl group having 1 to 4 carbon atoms, R₂ is selected from hydrogen, hydroxy or an alkoxy group having from 1 to 4 carbon atoms, and A is either -OH or 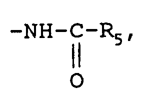 wherein R₅ is either hydrogen or an alkyl group having from 1 to about 4 carbon atoms, provided that if A is 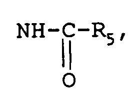 and R₁ and R₅ are both methyl and R₂ is hydrogen, both of R₃ and R₄ are not hydrogen.

15. Use according to claim 14, wherein the melatonin analog is N-acetyl serotonin, N-acetyl-5-hydroxy, 6-methoxy tryptamine, 6-hydroxy-melatonin, 5-hydroxytryptophol, or 5-methoxytryptophol.

16. Use of an estrogen for the manufacture of a medicament for alleviating or preventing menopausal or post-menopausal symptoms in a woman suffering from or susceptible to such symptoms by a method which comprises administering melatonin orally, intravenously or in the form of an implant on a daily basis in combination with said estrogen, optionally together with an androgen.

17. Use of an androgen for the manufacture of a medicament for alleviating or preventing menopausal or post-menopausal symptoms in a woman suffering from or susceptible to such symptoms by a method which comprises administering melatonin orally, intravenously or in the form of an implant on a daily basis in combination with said androgen, optionally together with an estrogen.

## Patentansprüche

1. Verwendung von Melatonin oder einem Melatoninanalogon zur Herstellung eines Medikaments zur Linderung oder Verhinderung von klimakterischen oder postklimakterischen Symptomen bei einer Frau, die an solchen Symptomen leidet oder zu solchen Symptomen neigt, durch ein Verfahren, das die Verabreichung dieses Melatonins oral, intravenös oder in Form eines Implantats auf einer täglichen Basis in Kombination mit einem oder mehreren Östrogenen, einem oder mehreren Androgenen oder sowohl einem Androgen als auch einem Östrogen umfaßt.

2. Verwendung gemäß Anspruch 1, bei dem das Melatonin in Kombination mit einem oder mehreren Östrogenen verabreicht wird und die Tagesdosis des Melatonins ausreicht, um die physiologische Toleranz der Frau für das verabreichte Östrogen zu erhöhen.

3. Verwendung gemäß Anspruch 2, wobei der Tagesdosisspiegel von Östrogen von 0,125 mg bis 15 mg, vorzugsweise von 0,3 mg bis 8 mg beträgt und der Tagesdosisspiegel von Melatonin 1 mg bis 2000 mg, vorzugsweise 30 mg bis 300 mg beträgt.

4. Verwendung gemäß Anspruch 2 oder 3, wobei das Östrogen ausgewählt wird aus Östron, Equilin, 17-α-Dihydroequilin, 17-α-Östradiol, Equilenin, 17-α-Dihydroequilenin, Ethinylöstradiol, Mestranol, Östradiol, Östriol, Diethylstilbestrol und Chinöstradiol, ggf. in Form eines Sulfatestersalzes.

5. Verwendung gemäß einem der Ansprüche 1 bis 4, wobei
i) eine Kombination von einem Östrogen und Melatonin täglich etwa 21 Tage lang verabreicht wird, gefolgt von einer Melatoninverabreichung täglich während etwa 7 Tagen in Abwesenheit eines Östrogens; oder
ii) eine Kombination von einem Östrogen und Melatonin täglich etwa 21 Tage lang verabreicht wird, gefolgt von etwa 7 Tagen ohne Melatonin- oder Östrogenverabreichung; oder
iii) ein Östrogen und Melatonin 60 bis 90 Tage lang verabreicht werden, dann sowohl das Östrogen wie das Melatonin abgesetzt werden, bis die klimakterischen oder postklimakterischen Symptome wieder auftreten.

6. Verwendung gemäß einem der Ansprüche 1 bis 5, wobei die Verabreichung durch intravenöse Injektion in einem physiologisch geeigneten Träger, durch Implantat oder oral erfolgt.

7. Verwendung gemäß einem der Ansprüche 1 bis 6, wobei ein Progestogen in Kombination mit dem Melatonin und einem oder mehreren Östrogenen verabreicht wird.

8. Verwendung gemäß Anspruch 7, wobei das Progestogen ausgewählt wird aus der Gruppe bestehend aus Norethindron, Norgestrel, Chlormadionon-acetat, Norethinodrel, Medoxyprogesteron-acetat, Megesterolacetat, Lynestrenol, Chingestron, Norethindron-acetat, Ethinodiol-acetat, Dimethisteron und Levonorgestrel.

9. Verwendung gemäß einem der Ansprüche 1 bis 8, wobei das Melatonin in Kombination mit wenigstens einem Androgen verabreicht wird.

10. Verwendung gemäß Anspruch 9, wobei das Androgen Methyltestosteron oder Fluoxymesteron ist.

11. Verwendung gemäß Anspruch 9 oder 10, wobei die tägliche Dosis des Androgens von 0,2 mg bis 15 mg beträgt und die tägliche Melatonindosis von 1 mg bis 2000 mg beträgt.

12. Verwendung von einem oder mehreren Östrogenen und/oder Melatonin oder einem Melatoninanalogon zur Herstellung von Medikamenten zur Bewirkung einer Östrogenergänzung bei einer Frau in oder nach der Menopause durch ein Verfahren, das die orale, intravenöse oder in Form eines Implantats erfolgende Verabreichung von wirksamen Dosen von einem oder mehreren Östrogenen in Kombination mit einer hinreichenden Menge Melatonin, um die physiologische Toleranz der Frau für Östrogen zu erhöhen, umfaßt.

13. Verwendung von Melatonin oder einem Melatoninanalogon zur Herstellung eines Medikaments zur Behandlung der Osteoporose, Verhinderung der Osteoporose, Behandlung der atrophischen Vaginitis, Behandlung der Kraurosis vulvae oder Behandlung von vasomotorischen Symptomen eines Östrogenmangels durch eine Methode, die die orale, intravenöse oder in Form eines Implantats erfolgende Verabreichung von Melatonin in Kombination mit wenigstens einem Östrogen und/oder wenigstens einem Androgen umfaßt.

14. Verwendung gemäß einem der Ansprüche 1 bis 13, bei dem das Melatoninanalogon die allgemeine Formel 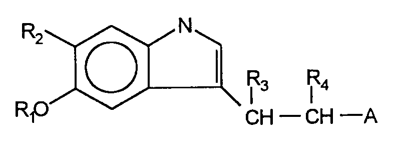 hat, wobei R₁, R₃ und R₄ jeweils Wasserstoff oder eine Alkylgruppe mit 1 bis 4 Kohlenstoffatomen sind, R₂ ausgewählt wird aus Wasserstoff, Hydroxy oder einer Alkoxygruppe mit 1 bis 4 Kohlenstoffatomen und A entweder -OH oder 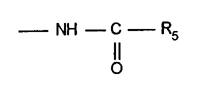 ist, wobei R₅ entweder Wasserstoff oder eine Alkylgruppe mit 1 bis etwa 4 Kohlenstoffatomen ist, mit der Maßgabe, daß wenn A 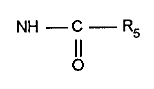 ist und R₁ und R₅ beide Methyl sind und R₂ Wasserstoff ist, nicht sowohl R₃ wie auch R₄ Wasserstoff sind.

15. Verwendung gemäß Anspruch 14, wobei das Melatoninanalogon N-Acetyl-serotonin, N-Acetyl-5-hydroxy-6-methoxy-tryptamin, 6-Hydroxy-melatonin, 5-Hydroxytryptophol oder 5-Methoxytryptophol ist.

16. Verwendung eines Östrogens zur Herstellung eines Medikaments zur Linderung oder Verhinderung von klimakterischen oder postklimakterischen Symptomen in einer Frau, die an solchen Symptomen leidet oder zu solchen Symptomen neigt, durch eine Methode, die die orale, intravenöse oder in Form eines Implantats erfolgende Verabreichung von Melatonin auf täglicher Basis in Kombination mit diesem Östrogen, ggf. zusammen mit einem Androgen, umfaßt.

17. Verwendung eines Androgens zur Herstellung eines Medikaments zur Linderung oder Verhinderung von klimakterischen oder postklimakterischen Symptomen bei einer Frau, die an solchen Symptomen leidet oder zu solchen Symptomen neigt, durch eine Methode, die die orale, intravenöse oder in Form eines Implantats erfolgende Verabreichung von Melatonin auf täglicher Basis in Kombination mit diesem Androgen, ggf. zusammen mit einem Östrogen, umfaßt.

## Revendications

1. Utilisation de la mélatonine ou d'un analogue de la mélatonine pour la fabrication d'un médicament destiné à réduire ou empêcher les symptômes liés à la période ménopausique ou post-ménopausique chez la femme souffrant de ou sensible à ces symptômes par un procédé qui comprend l'administration quotidienne de ladite mélatonine par voie orale, par voie intraveineuse ou sous la forme d'un implant en combinaison avec un ou plusieurs oestrogènes, un ou plusieurs androgènes ou à la fois un oestrogène et un androgène.

2. Utilisation selon la revendication 1, dans laquelle la mélatonine est administrée en combinaison avec un ou plusieurs oestrogènes et dans laquelle la dose quotidienne de mélatonine est suffisante pour augmenter chez la femme la tolérance physiologique envers l'oestrogène administré.

3. Utilisation selon la revendication 2, dans laquelle la dose quotidienne d'oestrogène est compris entre 0,125 mg et 15 mg, de préférence entre 0,3 mg et 8 mg, et la dose quotidienne de mélatonine est comprise entre 1 mg et 2000 mg, de préférence entre 30 mg et 300 mg.

4. Utilisation selon la revendication 2 ou 3, dans laquelle l'oestrogène est choisi parmi l'oestrone, l'équiline, le 17-α-dihydroéquiline, le 17-α-oestradiol, l'équilénine, le 17-α-dihydroéquilénine, l'éthinyloestradiol, le mestranol, l'oestradiol, l'oestriol, le diéthylstilbestrol et le quinoestradiol facultativement sous la forme d'un sel de sulfate ester.

5. Utilisation selon l'une quelconque des revendications 1 à 4, dans laquelle
i) une combinaison d'un oestrogène et de la mélatonine est administrée quotidiennement pendant environ 21 jours suivi de l'administration de mélatonine pendant environ 7 jours en l'absence d'un oestrogène; ou
ii) une combinaison d'un oestrogène et de la mélatonine est administrée quotidiennement pendant environ 21 jours suivi de l'administration sans mélatonine ou sans oestrogène pendant environ 7 jours; ou
iii) un oestrogène et la mélatonine sont administrés pendant 60 à 90 jours, puis à la fois l'oestrogène et la mélatonine sont maintenus jusqu'à ce que les symptômes liés à la période ménopausique ou postménopausique réapparaissent.

6. Utilisation selon l'une quelconque des revendications 1 à 5, dans laquelle le procédé d'administration est une injection par voie intraveineuse dans un support physiologiquement acceptable, un implant ou une administration par voie orale.

7. Utilisation selon l'une quelconque des revendications 1 à 6, dans laquelle un progestrogène est administré en combinaison avec la mélatonine et un ou plusieurs oestrogènes.

8. Utilisation selon la revendication 7, dans laquelle le progestrogène est choisi dans le groupe constitué de la noréthindrone, le norgestrel, la chlormadionone-acétate, le noréthynodrel, l'acétate de médoxyprogestérone, l'acétate de mégestérol, le lynestrénol, la quingestrone, l'acétate de noréthindrone, l'acétate d'éthynodiol, le diméthistérone, et le lévonorgestrel.

9. Utilisation selon l'une quelconque des revendications 1 à 8, dans laquelle la mélatonine est administrée en combinaison avec au moins un androgène.

10. Utilisation selon la revendication 9, dans laquelle l'androgène est la méthyltestostérone ou la fluoxymestérone.

11. Utilisation selon la revendication 9 ou 10, dans laquelle la dose quotidienne d'androgène est comprise entre 0,2 mg et 15 mg et la dose quotidienne de mélatonine est comprise entre 1 mg et 2000 mg.

12. Utilisation d'un ou plusieurs oestrogènes et/ou de la mélatonine ou d'un analogue de la mélatonine pour la fabrication de médicaments destinés à accroître la quantité d'oestrogène chez la femme en période ménopausique ou post-ménopausique par un procédé qui comprend l'administration par voie orale, par voie intraveineuse ou sous la forme d'un implant de doses d'un ou plusieurs oestrogènes en combinaison avec une quantité suffisante de mélatonine pour augmenter la tolérance physiologique envers l'oestrogène chez la femme.

13. Utilisation de la mélatonine ou d'un analogue de la mélatonine pour la fabrication d'un médicament destiné à traiter l'ostéoporose, d'empêcher l'ostéoporose, de traiter les vaginites atrophiques, de traiter les kraurosis vulvae ou de traiter les symptômes vasomoteurs de la déficience en oestrogène par un procédé qui comprend l'administration par voie orale, par voie intraveineuse ou sous la forme d'un implant de la mélatonine en combinaison avec au moins un oestrogène et/ou au moins un androgène.

14. Utilisation selon l'une quelconque des revendications 1 à 13, dans laquelle l'analogue de la mélatonine a la formule générale 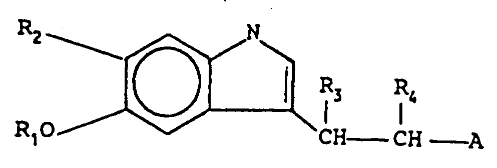 où R₁, R₃ et R₄, individuellement, sont l'hydrogène ou un groupe alcoyle ayant 1 à 4 atomes de carbone, R₂ est choisi parmi l'hydrogène, un groupe hydroxyle ou un groupe alcoxyle ayant 1 à 4 atomes de carbone, et A est soit -OH ou 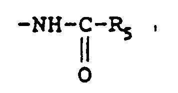 où R₅ est soit l'hydrogène ou un groupe alcoyle ayant 1 à environ 4 atomes de carbone, pourvu que si A est 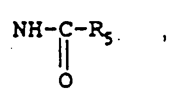 et R₁ et R₅ sont tous les deux un méthyle et R₂ est l'hydrogène, à la fois R₃ et R₄ ne soient pas l'hydrogène.

15. Utilisation selon la revendication 14, où l'analogue de la mélatonine est la sérotonine N-acétylique, la N-acétyl-5-hydroxy, 6-méthoxy tryptamine, la 6-hydroxy-mélatonine, le 5-hydroxytryptophol, ou le 5-méthoxytryptophol.

16. Utilisation d'un oestrogène pour la fabrication d'un médicament destiné à réduire ou à empêcher les symptômes de la période ménopausique ou post-ménopausique chez une femme souffrant de ou sensible à ces symptômes par un procédé qui comprend l'administration quotidienne de la mélatonine par voie orale, par voie intraveineuse ou sous la forme d'un implant en combinaison avec ledit oestrogène, facultativement avec un androgène.

17. Utilisation d'un androgène pour la fabrication d'un médicament destiné à réduire ou empêcher les symptômes liés à la période ménopausique ou post-ménopausique chez la femme souffrant de ou sensible à ces symptômes par un procédé qui comprend l'administration quotidienne de mélatonine par voie orale, par voie intraveineuse ou sous la forme d'un implant en combinaison avec ledit androgène, facultativement avec un oestrogène.
